# EUROPEAN PATENT APPLICATION

(11) **EP 3 296 314 A1**
(43) Date of publication of application: **21.03.2018**
(21) Application number: 16792700.3
(22) Date of filing: 10.05.2016
(51) Int. Cl.: C07K 1/113, A23J 3/00, C08F 20/38, C08F 20/60, C12N 9/96

(54) **PROTEIN AGGREGATION INHIBITOR**

(30) Priority: 11.05.2015 JP 2015096440
(71) Applicant: Osaka Organic Chemical Industry Ltd., Osaka-shi, Osaka 541-0052 (JP); Japan Advanced Institute of Science and Technology, Ishikawa 923-1292 (JP)
(72) Inventor: MATSUMURA, Kazuaki, Nomi-shi Ishikawa 923-1292 (JP); ROBIN, Rajan, Nomi-shi Ishikawa 923-1292 (JP); FURUKAWA, Tsuyoshi, Hakusan-shi Ishikawa 924-0057 (JP); MAEHARA, Kentaro, Hakusan-shi Ishikawa 924-0057 (JP); SARUWATARI, Yoshiyuki, Osaka-shi Osaka 541-0052 (JP)
(74) Representative: J A Kemp
(86) International application number: PCT/JP2016/063929
(87) International publication number: WO 2016/181975

(57) **Abstract**

Provided is a protein aggregation inhibitor capable of inhibiting aggregation of a protein even when the protein is heated, and further, capable of inhibiting a decrease in the protein activity even when the protein is heated, which characteristically contains a sulfobetaine polymer obtained by polymerizing monomer components containing sulfobetaine monomer represented by the formula (I): 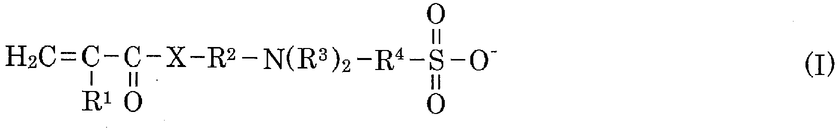 wherein R¹ is a hydrogen atom or a methyl group, R² is an alkylene group having a carbon number of 1 - 4, R³ is an alkyl group having a carbon number of 1 - 4, R⁴ is an alkylene group having a carbon number of 1 - 4, and X is an -NH- group or an - O- group.

## Description

### [Technical Field]

The present invention relates to a protein aggregation inhibitor. Since the protein aggregation inhibitor of the present invention can effectively prevent aggregation of protein even when the protein is heated, it is expected to be used, for example, for various applications where inhibition of proteins aggregation is desired such as preservative for enzyme, antibody drug, in vivo amyloid aggregation inhibitor and the like.

### [Background Art]

As a protein aggregation inhibitor capable of preventing aggregation of protein under acidic conditions, a protein aggregation inhibitor containing at least one kind of non-ionic surfactant selected from the group consisting of polyoxyethylene distyrenated phenyl ether, polyoxyethylene myristyl ether and polyoxyethylene(10) octylphenyl ether has been proposed (e.g., patent document 1).

Since the aforementioned protein aggregation inhibitor can inhibit, to some extent, aggregation of proteins under acidic conditions since it uses a surfactant.

However, for example, proteins such as egg yolk and egg white of chicken egg and the like are generally weak to heat, and show property of easy aggregation by heating, and the aforementioned protein aggregation inhibitor cannot sufficiently inhibit aggregation of proteins by heating.

### [Document List]

### [Patent document]

patent document 1: JP-A-2006-343201

### [SUMMARY OF THE INVENTION]

### [Problems to be Solved by the Invention]

The present invention has been made in view of the aforementioned prior art, and aims to provide a protein aggregation inhibitor which can inhibit aggregation of protein even when the protein is heated, and can further inhibit decrease in the protein activity even when the protein is heated.

### [Means of Solving the Problems]

The present invention relates to a protein aggregation inhibitor used for preventing protein aggregation and characteristically containing a sulfobetaine polymer obtained by polymerizing monomer components containing sulfobetaine monomer represented by the formula (I): 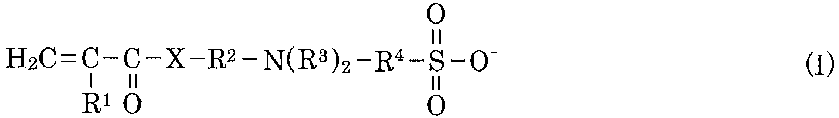 wherein R¹ is a hydrogen atom or a methyl group, R² is an alkylene group having a carbon number of 1 - 4, R³ is an alkyl group having a carbon number of 1 - 4, R⁴ is an alkylene group having a carbon number of 1 - 4, and X is an -NH- group or an - O- group.

### [Effect of the Invention]

According to the protein aggregation inhibitor of the present invention, a superior effect that aggregation of a protein can be inhibited even when the protein is heated, and further, a decrease in the protein activity can be inhibited even when the protein is heated is provided.

### [Description of Embodiments]

The protein aggregation inhibitor of the present invention is a protein aggregation inhibitor used to prevent aggregation of a protein, as mentioned above, and characteristically contains a sulfobetaine polymer obtained by polymerizing monomer components containing sulfobetaine monomer represented by the formula (I): 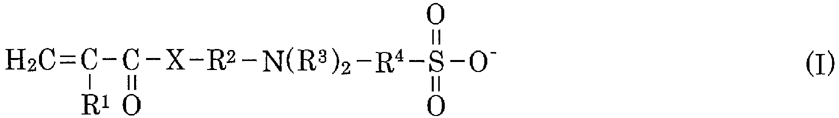 wherein R¹ is a hydrogen atom or a methyl group, R² is an alkylene group having a carbon number of 1 - 4, R³ is an alkyl group having a carbon number of 1 - 4, R⁴ is an alkylene group having a carbon number of 1 - 4, and X is an -NH- group or an - O- group.

In the sulfobetaine monomer represented by the formula (I), R¹ is a hydrogen atom or a methyl group.

R² is an alkylene group having a carbon number of 1 - 4. As the alkylene group having a carbon number of 1 - 4, a methylene group, an ethylene group, a propylene group and a butylene group can be mentioned. Of these groups, a methylene group, an ethylene group and a propylene group are preferable from the aspect that aggregation of a protein can be inhibited even when the protein is heated, and further, a decrease in the protein activity is inhibited even when the protein is heated.

R³ is an alkyl group having a carbon number of 1 - 4. As the alkyl group having a carbon number of 1 - 4, a methyl group, an ethyl group, a propyl group and a butyl group can be mentioned. Of these groups, a methyl group, an ethyl group and a propyl group are preferable, a methyl group and an ethyl group are more preferable, and a methyl group is further preferable from the aspect that aggregation of a protein can be inhibited even when the protein is heated, and further, a decrease in the protein activity is inhibited even when the protein is heated.

R⁴ is an alkylene group having a carbon number of 1 - 4. As the alkylene group having a carbon number of 1 - 4, a methylene group, an ethylene group, a propylene group and a butylene group can be mentioned. Of these groups, a methylene group, an ethylene group and a propylene group are preferable from the aspect that aggregation of a protein can be inhibited even when the protein is heated, and further, a decrease in the protein activity is inhibited even when the protein is heated.

X is an -NH- group or an -O- group. Of these groups, an -NH- group is preferable from the aspect that aggregation of a protein can be inhibited even when the protein is heated, and further, a decrease in the protein activity is inhibited even when the protein is heated.

Examples of the sulfobetaine monomer represented by the formula (I) include 3-[(3-(meth)acrylamidoalkyl)dialkylammonio]alkane-1-sulfonates such as 3-[(3-acrylamidomethyl)dimethylammonio]ethane-1-sulfonate, 3-[(3-methacrylamidomethyl)dimethylammonio]ethane-1-sulfonate, 3-[(3-acrylamidomethyl)diethylammonio]ethane-1-sulfonate, 3-[(3-methacrylamidomethyl)diethylammonio]ethane-1-sulfonate, 3-[(3-acrylamidomethyl)dipropylammonio]ethane-1-sulfonate, 3-[(3-methacrylamidomethyl)dipropylammonio]ethane-1-sulfonate, 3-[(3-acrylamidomethyl)dibutylammonio]ethane-1-sulfonate, 3-[(3-methacrylamidomethyl)dibutylammonio]ethane-1-sulfonate, 3-[(3-acrylamidoethyl)dimethylammonio]ethane-1-sulfonate, 3-[(3-methacrylamidoethyl)dimethylammonio]ethane-1-sulfonate, 3-[(3-acrylamidoethyl)diethylammonio]ethane-1-sulfonate, 3-[(3-methacrylamidoethyl)diethylammonio]ethane-1-sulfonate, 3-[(3-acrylamidoethyl)dipropylammonio]ethane-1-sulfonate, 3-[(3-methacrylamidoethyl)dipropylammonio]ethane-1-sulfonate, 3-[(3-acrylamidoethyl)dibutylammonio]ethane-1-sulfonate, 3-[(3-methacrylamidoethyl)dibutylammonio]ethane-1-sulfonate, 3-[(3-acrylamidopropyl)dimethylammonio]ethane-1-sulfonate, 3-[(3-methacrylamidopropyl)dimethylammonio]ethane-1-sulfonate, 3-[(3-acrylamidopropyl)diethylammonio]ethane-1-sulfonate, 3-[(3-methacrylamidopropyl)diethylammonio]ethane-1-sulfonate, 3-[(3-acrylamidopropyl)dipropylammonio]ethane-1-sulfonate, 3-[(3-methacrylamidopropyl)dipropylammonio]ethane-1-sulfonate, 3-[(3-acrylamidopropyl)dibutylammonio]ethane-1-sulfonate, 3-[(3-methacrylamidopropyl)dibutylammonio]ethane-1-sulfonate, 3-[(3-acrylamidobutyl)dimethylammonio]ethane-1-sulfonate, 3-[(3-methacrylamidobutyl)dimethylammonio]ethane-1-sulfonate, 3-[(3-acrylamidobutyl)diethylammonio]ethane-1-sulfonate, 3-[(3-methacrylamidobutyl)diethylammonio]ethane-1-sulfonate, 3-[(3-acrylamidobutyl)dipropylammonio]ethane-1-sulfonate, 3-[(3-methacrylamidobutyl)dipropylammonio]ethane-1-sulfonate, 3-[(3-acrylamidobutyl)dibutylammonio]ethane-1-sulfonate, 3-[(3-methacrylamidobutyl)dibutylammonio]ethane-1-sulfonate, 3-[(3-acrylamidomethyl)dimethylammonio]propane-1-sulfonate, 3-[(3-methacrylamidomethyl)dimethylammonio]propane-1-sulfonate, 3-[(3-acrylamidomethyl)diethylammonio]propane-1-sulfonate, 3-[(3-methacrylamidomethyl)diethylammonio]propane-1-sulfonate, 3-[(3-acrylamidomethyl)dipropylammonio]propane-1-sulfonate, 3-[(3-methacrylamidomethyl)dipropylammonio]propane-1-sulfonate, 3-[(3-acrylamidomethyl)dibutylammonio]propane-1-sulfonate, 3-[(3-methacrylamidomethyl)dibutylammonio]propane-1-sulfonate, 3-[(3-acrylamidoethyl)dimethylammonio]propane-1-sulfonate, 3-[(3-methacrylamidoethyl)dimethylammonio]propane-1-sulfonate, 3-[(3-acrylamidoethyl)diethylammonio]propane-1-sulfonate, 3-[(3-methacrylamidoethyl)diethylammonio]propane-1-sulfonate, 3-[(3-acrylamidoethyl)dipropylammonio]propane-1-sulfonate, 3-[(3-methacrylamidoethyl)dipropylammonio]propane-1-sulfonate, 3-[(3-acrylamidoethyl)dibutylammonio]propane-1-sulfonate, 3-[(3-methacrylamidoethyl)dibutylammonio]propane-1-sulfonate, 3-[(3-acrylamidopropyl)dimethylammonio]propane-1-sulfonate, 3-[(3-methacrylamidopropyl)dimethylammonio]propane-1-sulfonate, 3-[(3-acrylamidopropyl)diethylammonio]propane-1-sulfonate, 3-[(3-methacrylamidopropyl)diethylammonio]propane-1-sulfonate, 3-[(3-acrylamidopropyl)dipropylammonio]propane-1-sulfonate, 3-[(3-methacrylamidopropyl)dipropylammonio]propane-1-sulfonate, 3-[(3-acrylamidopropyl)dibutylammonio]propane-1-sulfonate, 3-[(3-methacrylamidopropyl)dibutylammonio]propane-1-sulfonate, 3-[(3-acrylamidobutyl)dimethylammonio]propane-1-sulfonate, 3-[(3-methacrylamidobutyl)dimethylammonio]propane-1-sulfonate, 3-[(3-acrylamidobutyl)diethylammonio]propane-1-sulfonate, 3-[(3-methacrylamidobutyl)diethylammonio]propane-1-sulfonate, 3-[(3-acrylamidobutyl)dipropylammonio]propane-1-sulfonate, 3-[(3-methacrylamidobutyl)dipropylammonio]propane-1-sulfonate, 3-[(3-acrylamidobutyl)dibutylammonio]propane-1-sulfonate, 3-[(3-methacrylamidobutyl)dibutylammonio]propane-1-sulfonate, 3-[(3-acrylamidomethyl)dimethylammonio]butane-1-sulfonate, 3-[(3-methacrylamidomethyl)dimethylammonio]butane-1-sulfonate, 3-[(3-acrylamidomethyl)diethylammonio]butane-1-sulfonate, 3-[(3-methacrylamidomethyl)diethylammonio]butane-1-sulfonate, 3-[(3-acrylamidomethyl)dipropylammonio]butane-1-sulfonate, 3-[(3-methacrylamidomethyl)dipropylammonio]butane-1-sulfonate, 3-[(3-acrylamidomethyl)dibutylammonio]butane-1-sulfonate, 3-[(3-methacrylamidomethyl)dibutylammonio]butane-1-sulfonate, 3-[(3-acrylamidoethyl)dimethylammonio]butane-1-sulfonate, 3-[(3-methacrylamidoethyl)dimethylammonio]butane-1-sulfonate, 3-[(3-acrylamidoethyl)diethylammonio]butane-1-sulfonate, 3-[(3-methacrylamidoethyl)diethylammonio]butane-1-sulfonate, 3-[(3-acrylamidoethyl)dipropylammonio]butane-1-sulfonate, 3-[(3-methacrylamidoethyl)dipropylammonio]butane-1-sulfonate, 3-[(3-acrylamidoethyl)dibutylammonio]butane-1-sulfonate, 3-[(3-methacrylamidoethyl)dibutylammonio]butane-1-sulfonate, 3-[(3-acrylamidopropyl)dimethylammonio]butane-1-sulfonate, 3-[(3-methacrylamidopropyl)dimethylammonio]butane-1-sulfonate, 3-[(3-acrylamidopropyl)diethylammonio]butane-1-sulfonate, 3-[(3-methacrylamidopropyl)diethylammonio]butane-1-sulfonate, 3-[(3-acrylamidopropyl)dipropylammonio]butane-1-sulfonate, 3-[(3-methacrylamidopropyl)dipropylammonio]butane-1-sulfonate, 3-[(3-acrylamidopropyl)dibutylammonio]butane-1-sulfonate, 3-[(3-methacrylamidopropyl)dibutylammonio]butane-1-sulfonate, 3-[(3-acrylamidobutyl)dimethylammonio]butane-1-sulfonate, 3-[(3-methacrylamidobutyl)dimethylammonio]butane-1-sulfonate, 3-[(3-acrylamidobutyl)diethylammonio]butane-1-sulfonate, 3-[(3-methacrylamidobutyl)diethylammonio]butane-1-sulfonate, 3-[(3-acrylamidobutyl)dipropylammonio]butane-1-sulfonate, 3-[(3-methacrylamidobutyl)dipropylammonio]butane-1-sulfonate, 3-[(3-acrylamidobutyl)dibutylammonio]butane-1-sulfonate, 3-[(3-methacrylamidobutyl)dibutylammonio]butane-1-sulfonate and the like;
3-[(3-(meth)acryloyloxyalkyl)dialkylammonio]alkane-1-sulfonates such as 3-[(3-acryloyloxymethyl)dimethylammonio]ethane-1-sulfonate, 3-[(3-methacryloyloxymethyl)dimethylammonio]ethane-1-sulfonate, 3-[(3-acryloyloxymethyl)diethylammonio]ethane-1-sulfonate, 3-[(3-methacryloyloxymethyl)diethylammonio]ethane-1-sulfonate, 3-[(3-acryloyloxymethyl)dipropylammonio]ethane-1-sulfonate, 3-[(3-methacryloyloxymethyl)dipropylammonio]ethane-1-sulfonate, 3-[(3-acryloyloxymethyl)dibutylammonio]ethane-1-sulfonate, 3-[(3-methacryloyloxymethyl)dibutylammonio]ethane-1-sulfonate, 3-[(3-acryloyloxyethyl)dimethylammonio]ethane-1-sulfonate, 3-[(3-methacryloyloxyethyl)dimethylammonio]ethane-1-sulfonate, 3-[(3-acryloyloxyethyl)diethylammonio]ethane-1-sulfonate, 3-[(3-methacryloyloxyethyl)diethylammonio]ethane-1-sulfonate, 3-[(3-acryloyloxyethyl)dipropylammonio]ethane-1-sulfonate, 3-[(3-methacryloyloxyethyl)dipropylammonio]ethane-1-sulfonate, 3-[(3-acryloyloxyethyl)dibutylammonio]ethane-1-sulfonate, 3-[(3-methacryloyloxyethyl)dibutylammonio]ethane-1-sulfonate, 3-[(3-acryloyloxypropyl)dimethylammonio]ethane-1-sulfonate, 3-[(3-methacryloyloxypropyl)dimethylammonio]ethane-1-sulfonate, 3-[(3-acryloyloxypropyl)diethylammonio]ethane-1-sulfonate, 3-[(3-methacryloyloxypropyl)diethylammonio]ethane-1-sulfonate, 3-[(3-acryloyloxypropyl)dipropylammonio]ethane-1-sulfonate, 3-[(3-methacryloyloxypropyl)dipropylammonio]ethane-1-sulfonate, 3-[(3-acryloyloxypropyl)dibutylammonio]ethane-1-sulfonate, 3-[(3-methacryloyloxypropyl)dibutylammonio]ethane-1-sulfonate, 3-[(3-acryloyloxybutyl)dimethylammonio]ethane-1-sulfonate, 3-[(3-methacryloyloxybutyl)dimethylammonio]ethane-1-sulfonate, 3-[(3-acryloyloxybutyl)diethylammonio]ethane-1-sulfonate, 3-[(3-methacryloyloxybutyl)diethylammonio]ethane-1-sulfonate, 3-[(3-acryloyloxybutyl)dipropylammonio]ethane-1-sulfonate, 3-[(3-methacryloyloxybutyl)dipropylammonio]ethane-1-sulfonate, 3-[(3-acryloyloxybutyl)dibutylammonio]ethane-1-sulfonate, 3-[(3-methacryloyloxybutyl)dibutylammonio]ethane-1-sulfonate, 3-[(3-acryloyloxymethyl)dimethylammonio]propane-1-sulfonate, 3-[(3-methacryloyloxymethyl)dimethylammonio]propane-1-sulfonate, 3-[(3-acryloyloxymethyl)diethylammonio]propane-1-sulfonate, 3-[(3-methacryloyloxymethyl)diethylammonio]propane-1-sulfonate, 3-[(3-acryloyloxymethyl)dipropylammonio]propane-1-sulfonate, 3-[(3-methacryloyloxymethyl)dipropylammonio]propane-1-sulfonate, 3-[(3-acryloyloxymethyl)dibutylammonio]propane-1-sulfonate, 3-[(3-methacryloyloxymethyl)dibutylammonio]propane-1-sulfonate, 3-[(3-acryloyloxyethyl)dimethylammonio]propane-1-sulfonate, 3-[(3-methacryloyloxyethyl)dimethylammonio]propane-1-sulfonate, 3-[(3-acryloyloxyethyl)diethylammonio]propane-1-sulfonate, 3-[(3-methacryloyloxyethyl)diethylammonio]propane-1-sulfonate, 3-[(3-acryloyloxyethyl)dipropylammonio]propane-1-sulfonate, 3-[(3-methacryloyloxyethyl)dipropylammonio]propane-1-sulfonate, 3-[(3-acryloyloxyethyl)dibutylammonio]propane-1-sulfonate, 3-[(3-methacryloyloxyethyl)dibutylammonio]propane-1-sulfonate, 3-[(3-acryloyloxypropyl)dimethylammonio]propane-1-sulfonate, 3-[(3-methacryloyloxypropyl)dimethylammonio]propane-1-sulfonate, 3-[(3-acryloyloxypropyl)diethylammonio]propane-1-sulfonate, 3-[(3-methacryloyloxypropyl)diethylammonio]propane-1-sulfonate, 3-[(3-acryloyloxypropyl)dipropylammonio]propane-1-sulfonate, 3-[(3-methacryloyloxypropyl)dipropylammonio]propane-1-sulfonate, 3-[(3-acryloyloxypropyl)dibutylammonio]propane-1-sulfonate, 3-[(3-methacryloyloxypropyl)dibutylammonio]propane-1-sulfonate, 3-[(3-acryloyloxybutyl)dimethylammonio]propane-1-sulfonate, 3-[(3-methacryloyloxybutyl)dimethylammonio]propane-1-sulfonate, 3-[(3-acryloyloxybutyl)diethylammonio]propane-1-sulfonate, 3-[(3-methacryloyloxybutyl)diethylammonio]propane-1-sulfonate, 3-[(3-acryloyloxybutyl)dipropylammonio]propane-1-sulfonate, 3-[(3-methacryloyloxybutyl)dipropylammonio]propane-1-sulfonate, 3-[(3-acryloyloxybutyl)dibutylammonio]propane-1-sulfonate, 3-[(3-methacryloyloxybutyl)dibutylammonio]propane-1-sulfonate, 3-[(3-acryloyloxymethyl)dimethylammonio]butane-1-sulfonate, 3-[(3-methacryloyloxymethyl)dimethylammonio]butane-1-sulfonate, 3-[(3-acryloyloxymethyl)diethylammonio]butane-1-sulfonate, 3-[(3-methacryloyloxymethyl)diethylammonio]butane-1-sulfonate, 3-[(3-acryloyloxymethyl)dipropylammonio]butane-1-sulfonate, 3-[(3-methacryloyloxymethyl)dipropylammonio]butane-1-sulfonate, 3-[(3-acryloyloxymethyl)dibutylammonio]butane-1-sulfonate, 3-[(3-methacryloyloxymethyl)dibutylammonio]butane-1-sulfonate, 3-[(3-acryloyloxyethyl)dimethylammonio]butane-1-sulfonate, 3-[(3-methacryloyloxyethyl)dimethylammonio]butane-1-sulfonate, 3-[(3-acryloyloxyethyl)diethylammonio]butane-1-sulfonate, 3-[(3-methacryloyloxyethyl)diethylammonio]butane-1-sulfonate, 3-[(3-acryloyloxyethyl)dipropylammonio]butane-1-sulfonate, 3-[(3-methacryloyloxyethyl)dipropylammonio]butane-1-sulfonate, 3-[(3-acryloyloxyethyl)dibutylammonio]butane-1-sulfonate, 3-[(3-methacryloyloxyethyl)dibutylammonio]butane-1-sulfonate, 3-[(3-acryloyloxypropyl)dimethylammonio]butane-1-sulfonate, 3-[(3-methacryloyloxypropyl)dimethylammonio]butane-1-sulfonate, 3-[(3-acryloyloxypropyl)diethylammonio]butane-1-sulfonate, 3-[(3-methacryloyloxypropyl)diethylammonio]butane-1-sulfonate, 3-[(3-acryloyloxypropyl)dipropylammonio]butane-1-sulfonate, 3-[(3-methacryloyloxypropyl)dipropylammonio]butane-1-sulfonate, 3-[(3-acryloyloxypropyl)dibutylammonio]butane-1-sulfonate, 3-[(3-methacryloyloxypropyl)dibutylammonio]butane-1-sulfonate, 3-[(3-acryloyloxybutyl)dimethylammonio]butane-1-sulfonate, 3-[(3-methacryloyloxybutyl)dimethylammonio]butane-1-sulfonate, 3-[(3-acryloyloxybutyl)diethylammonio]butane-1-sulfonate, 3-[(3-methacryloyloxybutyl)diethylammonio]butane-1-sulfonate, 3-[(3-acryloyloxybutyl)dipropylammonio]butane-1-sulfonate, 3-[(3-methacryloyloxybutyl)dipropylammonio]butane-1-sulfonate, 3-[(3-acryloyloxybutyl)dibutylammonio]butane-1-sulfonate, 3-[(3-methacryloyloxybutyl)dibutylammonio]butane-1-sulfonate and the like; and the like. However, the present invention is not limited to these exemplifications alone. These sulfobetaine monomers may each be used alone, or two or more kinds may be used in combination.

In the present invention, "(meth)acrylamide" means acrylamide or methacrylamide, acrylamide and methacrylamide may each be used alone, or may be used in combination. The "(meth)acryloyloxy" means acryloyloxy or methacryloyloxy, and acryloyloxy and methacryloyloxy may each be used alone, or may be used in combination. The "(meth)acrylate" means acrylate or methacrylate, and acrylate and methacrylate may each be used alone, or may be used in combination. The "(meth)acrylic acid" means acrylic acid or methacrylic acid, and acrylic acid and methacrylic acid may each be used alone, or may be used in combination.

The content of the sulfobetaine monomer represented by the formula (I) in monomer components varies depending on the use of the protein aggregation inhibitor of the present invention and cannot be decided unconditionally. From the aspect that aggregation of a protein can be inhibited even when the protein is heated, and further, a decrease in the protein activity is inhibited even when the protein is heated, the content is preferably not less than 50 mass %, more preferably not less than 60 mass %, and the upper limit thereof is 100 mass %. Therefore, the sulfobetaine polymer to be used in the protein aggregation inhibitor of the present invention may be a homopolymer of sulfobetaine monomers represented by the formula (I)

In the present invention, in the monomer components, a water-soluble monomer can be used as a monomer other than a sulfobetaine monomer represented by the formula (I).

The water-soluble monomer means a monomer showing dissolution property of not less than 50 g in 100 g of water at 25°C. Examples of the water-soluble monomer include (meth)acrylamide, N-vinylpyrrolidone, (meth)acrylonitrile, 2-hydroxyethyl (meth)acrylate, 2-hydroxypropyl (meth)acrylate, 4-hydroxybutyl (meth)acrylate, polyethylene glycol (meth)acrylate, methoxypolyethylene glycol (meth)acrylate, ethoxypolyethylene glycol (meth)acrylate, polyethylene glycol di(meth)acrylate, N-(meth)acrylmorpholide, N,N-dimethyl(meth)acrylamide, N-methoxymethyl(meth)acrylamide, N-hydroxymethyl(meth)acrylamide, N-hydroxyethyl(meth)acrylamide, 2-hydroxyethyl vinyl ether, N,N'-dimethyl(meth)acrylamide, N,N'-diethyl(meth)acrylamide, N-monomethyl(meth)acrylamide, N-monoethyl(meth)acrylamide and the like. However, the present invention is not limited to these exemplifications alone. These water-soluble monomers may each be used alone, or two or more kinds may be used in combination. Of these water-soluble monomers, (meth)acrylamide, N-vinylpyrrolidone and (meth)acrylonitrile are preferable, (meth)acrylamide and N-vinylpyrrolidone are more preferable, from the aspect that aggregation of a protein can be inhibited even when the protein is heated, and further, a decrease in the protein activity is inhibited even when the protein is heated.

The content of the water-soluble monomer in monomer components is preferably not more than 50 mass %, more preferably not more than 40 mass %, from the aspect that aggregation of a protein can be inhibited even when the protein is heated, and further, a decrease in the protein activity is inhibited even when the protein is heated, and the lower limit thereof is 0 mass %.

The monomer component may contain a water-insoluble monomer as long as the object of the present invention is not inhibited. The water-insoluble monomer means a monomer showing dissolution property of less than 50 g in 100 g of water at 25°C.

Examples of the water-insoluble monomer include alkyl (meth)acrylate, alkoxy group-containing (meth)acrylate, alicyclic group-containing (meth)acrylate, aryl group-containing (meth)acrylate, aromatic monomer other than an aryl group-containing (meth)acrylate and the like. However, the present invention is not limited to these exemplifications alone. These water-insoluble monomers may each be used alone, or two or more kinds may be used in combination.

Examples of the alkyl (meth)acrylate include methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, sec-butyl (meth)acrylate, n-pentyl (meth)acrylate, n-hexyl (meth)acrylate, n-octyl (meth)acrylate, isooctyl (meth)acrylate, n-nonyl (meth)acrylate, isononyl (meth)acrylate, 2-ethylhexyl (meth)acrylate, decyl (meth)acrylate, dodecyl (meth)acrylate, tridecyl (meth)acrylate, tetradecyl (meth)acrylate, hexadecyl (meth)acrylate, octadecyl (meth)acrylate, isostearyl (meth)acrylate, eicosyl (meth)acrylate, behenyl (meth)acrylate, tetrahydrofurfuryl (meth)acrylate and the like. However, the present invention is not limited to these exemplifications alone. These alkyl (meth)acrylates may each be used alone, or two or more kinds may be used in combination.

Examples of the alkoxy group-containing (meth)acrylate include 2-methoxyethyl (meth)acrylate, 2-ethoxyethyl (meth)acrylate, methoxyethylene glycol (meth)acrylate, ethylcarbitol (meth)acrylate and the like. However, the present invention is not limited to these exemplifications alone. These alkoxy group-containing (meth)acrylates may each be used alone, or two or more kinds may be used in combination.

Examples of the alicyclic group-containing (meth)acrylate include cyclohexyl (meth)acrylate, tert-butylcyclohexyl (meth)acrylate, isobornyl (meth)acrylate, dicyclopentanyl (meth)acrylate, dicyclopentenyl (meth)acrylate, adamantyl (meth)acrylate, dicyclopentenyl (meth)acrylate, cyclohexyl (meth)acrylate and the like. However, the present invention is not limited to these exemplifications alone. These alicyclic group-containing (meth)acrylates may each be used alone, or two or more kinds may be used in combination.

Examples of the aryl group-containing (meth)acrylate include (meth)acrylates having an aryl group containing a carbon number of 6 - 15, such as benzyl (meth)acrylate, phenoxyethyl (meth)acrylate and the like, and the like. However, the present invention is not limited to these exemplifications alone. These aryl group-containing (meth)acrylates may each be used alone, or two or more kinds may be used in combination.

Examples of the aromatic monomer other than an aryl group-containing (meth)acrylate include styrene, α-methylstyrene and the like. However, the present invention is not limited to these exemplifications alone. These aromatic monomers may each be used alone, or two or more kinds may be used in combination.

Examples of a method of polymerizing monomer components include bulk polymerization method, solution polymerization method, emulsion polymerization method, suspension polymerization method and the like. However, the present invention is not limited to these exemplifications alone. Of these polymerization methods, the solution polymerization method is preferable.

When monomer components are polymerized by the solution polymerization method, for example, the monomer components can be polymerized by dissolving the monomer components in an aqueous solvent, and adding a polymerization initiator to the obtained solution while stirring the solution. Alternatively, the monomer components can be polymerized by dissolving a polymerization initiator in an aqueous solvent, and adding monomer components to the obtained solution while stirring the solution.

The aqueous solvent is water or a mixed solvent of water and a hydrophilic organic solvent other than water. The content of water in the aqueous solvent is generally not less than 50 mass %, and the upper limit thereof is 100 mass %.

Examples of the hydrophilic organic solvent include monovalent aliphatic alcohols having a carbon number of 1 - 4 such as methyl alcohol, ethyl alcohol, propyl alcohol and the like; ketones such as acetone, methyl ethyl ketone and the like; ethers such as tetrahydrofuran, dioxane, diglyme and the like; amides such as N,N-dimethylformamide, N,N-dimethylacetamide, N-methyl-2-pyrrolidone, 1,3-dimethyl-2-imidazolidinone and the like; sulfur-containing organic solvents such as dimethyl sulfoxide, sulfolane and the like, and the like. However, the present invention is not limited to these exemplifications alone. These hydrophilic organic solvents may each be used alone, or two or more kinds may be used in combination. Of these hydrophilic organic solvents, a monovalent aliphatic alcohol having a carbon number of 1 - 4 is preferable, methyl alcohol, ethyl alcohol and propyl alcohol are more preferably, and methyl alcohol and ethyl alcohol are further preferable, from the aspect that aggregation of a protein can be inhibited even when the protein is heated, and further, a decrease in the protein activity is inhibited even when the protein is heated.

While the amount of the aqueous solvent is not particularly limited, it is generally preferably 50 - 400 parts by mass, more preferably 100 - 350 parts by mass, per 100 parts by mass of the monomer component.

When the monomer components are polymerized, a polymerization initiator is preferably used. Examples of the polymerization initiator include azobisisobutyronitrile, azoisobutyronitrile, azo methyl isobutyrate, azobisdimethylvaleronitrile, benzoyl peroxide, potassium persulfate, ammonium persulfate, benzophenone derivative, phosphineoxide derivative, benzoketone derivative, phenyl thioether derivative, azide derivative, diazo derivative, disulfide derivative and the like. However, the present invention is not limited to these exemplifications alone. These polymerization initiators may each be used alone, or two or more kinds may be used in combination. While the amount of the polymerization initiator is not particularly limited, it is generally preferably about 0.05 - 20 parts by mass per 100 parts by mass of the monomer component.

In the present invention, moreover, a chain transfer agent may also be used to adjust the molecular weight when the monomer components are polymerized. The chain transfer agent can be generally used by mixing with the monomer components. Examples of the chain transfer agent include mercaptan group-containing compounds such as 2-(dodecylthiocarbonothioylthio)-2-methylpropionic acid, 2-(dodecylthiocarbonothioylthio)propionic acid, methyl 2-(dodecylthiocarbonothioylthio)-2-methylpropionate, 2-(dodecylthiocarbonothioylthio)-2-methylpropionic acid 3-azido-1-propanol ester, 2-(dodecylthiocarbonothioylthio)-2-methylpropionic acid pentafluorophenyl ester, lauryl mercaptan, dodecyl mercaptan, thioglycerol and the like, inorganic salts such as sodium hypophosphite, sodium bisulfite and the like, and the like. However, the present invention is not limited to these exemplifications alone. These chain transfer agents may each be used alone, or two or more kinds may be used in combination. While the amount of the chain transfer agent is not particularly limited, it may be generally about 0.01 - 10 parts by mass per 100 parts by mass of the monomer component.

The polymerization reaction temperature and atmosphere when the monomer components are polymerized are not particularly limited. Generally, the polymerization reaction temperature is about 50 - 120°C. The atmosphere at the time of polymerization reaction is preferably, for example, inert gas atmosphere such as nitrogen gas and the like. While the polymerization reaction time of the monomer component cannot be decided unconditionally since it varies depending on the temperature of polymerization reaction and the like, it is generally about 3 - 20 hr.

A sulfobetaine polymer can be obtained by polymerizing monomer components as mentioned above.

The weight-average molecular weight of the sulfobetaine polymer is preferably 3000 - 100000, more preferably 5000 - 50000, from the aspect that aggregation of a protein can be inhibited even when the protein is heated, and further, a decrease in the protein activity is inhibited even when the protein is heated. The weight-average molecular weight of the sulfobetaine polymer is a value measured based on the method described in the following Examples.

When a monomer component is prepared by solution polymerization in the presence of an aqueous solvent, the reaction solution containing the resulting sulfobetaine polymer can be used as it is as a protein aggregation inhibitor. Where necessary, an aqueous solvent may be added to the aforementioned reaction solution such that the protein aggregation inhibitor contains the sulfobetaine polymer at a desired content, or the aqueous solvent contained in the reaction solution may be evaporated.

While the content of the sulfobetaine polymer (solid content) in the protein aggregation inhibitor of the present invention varies depending on the use of the protein aggregation inhibitor and the like, and cannot be decided unconditionally, it is generally about 3 - 80 mass %. The content of the sulfobetaine polymer (solid content) in the protein aggregation inhibitor of the present invention can be easily adjusted by adding a solvent to the protein aggregation inhibitor or evaporating the solvent contained in the protein aggregation inhibitor.

In addition, the protein aggregation inhibitor of the present invention may contain an additive and the like according to the use object of the protein aggregation inhibitor of the present invention, as long as the object of the present invention is not inhibited.

As explained above, since the protein aggregation inhibitor of the present invention can inhibit aggregation of a protein even when the protein is heated, and further, it can inhibit a decrease in the protein activity even when the protein is heated, it is expected to be used, for example, for various applications where inhibition of proteins aggregation is desired such as preservative for enzyme, antibody drug, in vivo amyloid aggregation inhibitor and the like.

### [Examples]

The present invention is explained in more detail in the following by referring to Examples. However, the present invention is not limited to the Examples alone.

### Example 1

In a 1 L flask provided with a stirring rod, a dimroth, a thermometer and a nitrogen gas introducing tube were placed 3-[(3-acylamidopropyl)dimethyltinmonio]propane-1-sulfonate (3.925 g), water (15 g), methyl alcohol (45 g) and 2-(dodecylthiocarbonothioylthio)-2-methylpropionic acid (0.2187 g), the contents of the flask were heated to 70°C while introducing a nitrogen gas into the flask, and the mixture was stirred. Thereafter, azobisisobutyronitrile (0.1947 g) as a polymerization initiator was added into the flask under stirring, and the monomer components in the flask were polymerized for 6 hr to give a sulfobetaine polymer solution.

The contents of the flask were cooled to room temperature, taken out from the flask, and the weight-average molecular weight of the obtained sulfobetaine polymer was by gel permeation chromatography (hereinafter to be referred to as GPC) analysis apparatus [HLC8220 GPC manufactured by Tosoh Corporation]. As a result, the weight-average molecular weight (based on polystyrene) of the obtained polymer was 5500. When the weight-average molecular weight of the obtained sulfobetaine polymer was examined by GPC, 100 mM aqueous sodium bromide solution was used as a developing solution.

Using the sulfobetaine polymer solution obtained above as a protein aggregation inhibitor, a protein aggregation inhibitory effect by the protein aggregation inhibitor was examined based on the following method.

Lysozyme (PBS) was added at a proportion of 3 mg per 1 mL protein aggregation inhibitor. The obtained solution was visually observed to find no aggregation of lysozyme.

Then, the solution obtained in the above was heated at 90°C for 30 min, and cooled to room temperature. The cooled solution was visually observed to find no aggregation of lysozyme.

### Comparative Example 1

Lysozyme (PBS) was added at a proportion of 3 mg per 1 mL water. The obtained aqueous solution was visually observed to find no aggregation of lysozyme.

Then, the aqueous solution obtained in the above was heated at 90°C for 30 min, and cooled to room temperature. The cooled solution was visually observed to find aggregation of lysozyme.

### Comparative Example 2

3-[(3-Acylamidopropyl)dimethyltinmonio]propane-1-sulfonate was dissolved in water to give an aqueous solution having a content of 3-[(3-acylamidopropyl)dimethyltinmonio]propane-1-sulfonate of about 5 mass %. The aqueous solution obtained above was used as a protein aggregation inhibitor. Lysozyme (PBS) was added at a proportion of 3 mg per 1 mL protein aggregation inhibitor. The obtained solution was visually observed to find no aggregation of lysozyme.

Then, the aqueous solution obtained in the above was heated at 90°C for 30 min, and cooled to room temperature. The cooled solution was visually observed to find aggregation of lysozyme.

From the above results, it is clear that lysozyme aggregates when the protein aggregation inhibitor obtained in Example 1 is absent and when lysozyme is heated using 3-[(3-acylamidopropyl)dimethyltinmonio]propane-1-sulfonate, which is a starting material monomer used for the preparation of a sulfobetaine polymer, whereas when lysozyme is heated in the presence of the protein aggregation inhibitor obtained in Example 1, aggregation of lysozyme is effectively inhibited even when heated.

### Experimental Example 1

Using the aqueous solution obtained in Comparative Example 1, the aqueous solution (2 mL) and the thioflavin T solution (PBS16 µg/mL) (100 µL) were mixed, the fluorescence intensity of the mixed solution (solution temperature: about 25°C) obtained above was measured at an excitation wavelength of 440 nm and an emission wavelength of 480 nm by using part number: T3516 manufactured by Sigma-Aldrich, and the basal fluorescence intensity was determined based on the formula: [basal fluorescence intensity]=[fluorescence intensity at emission wavelength]÷[fluorescence intensity at excitation wavelength] .

Then, using a solution added with lysozyme (PBS) obtained Example 1, the solution (2 mL) and thioflavin T solution (PBS16 µg/mL) (100 µL) were mixed. Using the obtained mixed solution (solution temperature: about 25°C) and in the same manner as above, the fluorescence intensity was determined, from which the ratio of the fluorescence intensity relative to the basal fluorescence intensity was determined to find a fluorescence intensity ratio of 19.2%.

### Comparative Experimental Example 1

In the same manner as in Experimental Example 1 except that a solution (solution temperature: about 25°C) added with lysozyme (PBS) obtained in Comparative Example 2 was used instead of the solution added with lysozyme (PBS) obtained in Example 1, the experiment was performed. As a result, the ratio of fluorescence intensity relative to the basal fluorescence intensity was 57.0% when the solution added with lysozyme (PBS) obtained in Comparative Example 2 was used.

### Comparative Experimental Example 2

In the same manner as in Experimental Example 1 except that a solution (solution temperature: about 25°C) added with arginine in an amount equal to the sulfobetaine polymer used in Example 1 was used instead of the sulfobetaine polymer, the experiment was performed. As a result, the ratio of fluorescence intensity relative to the basal fluorescence intensity was 66.3% when the solution added with arginine was used.

### Comparative Experimental Example 3

In the same manner as in Experimental Example 1 except that a solution (solution temperature: about 25°C) added with 3-(ethyldimethylammonio)propane-1-sulfonate (part number: NDSB195 manufactured by Anatrace) in an amount equal to the sulfobetaine polymer used in Example 1 was used instead of the sulfobetaine polymer, the experiment was performed. As a result, the ratio of fluorescence intensity relative to the basal fluorescence intensity was 45.0% when the solution added with 3-(ethyldimethylammonio)propane-1-sulfonate was used.

### Comparative Experimental Example 4

In the same manner as in Experimental Example 1 except that a solution (solution temperature: about 25°C) added with 1-(3-sulfopropyl)pyridium (part number: NDSB201 manufactured by Anatrace) in an amount equal to the sulfobetaine polymer used in Example 1 was used instead of the sulfobetaine polymer, the experiment was performed. As a result, the ratio of fluorescence intensity relative to the basal fluorescence intensity was 98.8% when the solution added with 1-(3-sulfopropyl)pyridium was used.

### Comparative Experimental Example 5

In the same manner as in Experimental Example 1 except that a solution (solution temperature: about 25°C) added with 3-[(2-hydroxyethyl)dimethylammonio]propane-1-sulfonenate (part number: NDSB211 manufactured by Anatrace) in an amount equal to the sulfobetaine polymer used in Example 1 was used instead of the sulfobetaine polymer, the experiment was performed. As a result, the ratio of fluorescence intensity relative to the basal fluorescence intensity was 63.8% when the solution added with 3-[(2-hydroxyethyl)dimethylammonio]propane-1-sulfonenate was used.

### Comparative Experimental Example 6

In the same manner as in Experimental Example 1 except that a solution (solution temperature: about 25°C) added with 3-benzyldimethylammonio)propane-1-sulfonate (part number: NDSB256 manufactured by Anatrace) in an amount equal to the sulfobetaine polymer used in Example 1 was used instead of the sulfobetaine polymer, the experiment was performed. As a result, the ratio of fluorescence intensity relative to the basal fluorescence intensity was 62.0% when the solution added with 3-benzyldimethylammonio)propane-1-sulfonate was used.

From the results of the aforementioned Experimental Example 1 and Comparative Experimental Examples 1 - 6, it is clear that Experimental Example 1 can effectively inhibit aggregation of protein, since the ratio of fluorescence intensity relative to the basal fluorescence intensity is low due to the use of a sulfobetaine polymer.

### Experimental Example 2

Lysozyme (PBS) was added at a proportion of 3 mg per 1 mL protein aggregation inhibitor obtained in Example 1. The obtained solution was visually observed to find no aggregation of lysozyme.

Then, the solution obtained in the above was heated at 90°C for 30 min, and cooled to room temperature. The cooled solution was visually observed to find no aggregation of lysozyme.

Then, the time-course changes in the absorbance at wavelength 600 nm of the aforementioned solution after heating at 90°C for 30 min was examined by a spectrophotometer [SHIMADZU CORPORATION, part number: UVPC1600]. As a result, the absorbance of a solution after heating at 90°C for 30 min and cooling to room temperature was 0.85, and the absorbance of a solution after lapse of 6 min from cooling to room temperature was 0.8.

On the other hand, an aqueous lysozyme solution was prepared by adding lysozyme (PBS) at a proportion of 3 mg per 1 mL water.

Using the aqueous lysozyme solution obtained above as it was without heating, time course changes in the absorbance at wavelength 600 nm were examined in the same manner as above. As a result, the initial absorbance of the aqueous lysozyme solution was 0.73, and the absorbance of the solution after lapse of 6 min was 0.48.

From the above results, it is clear that the activity of protein markedly decreases when an aqueous solution of the protein is left standing at room temperature, whereas when the protein aggregation inhibitor obtained in Example 1 is used, a decrease in the protein activity is inhibited even though the protein is heated.

### Experimental Example 3

Egg white was taken out by partly removing the eggshell of a chicken egg, the egg white thus taken out was placed in a petri dish, and the protein aggregation inhibitor obtained in Example 1 was attached to the whole egg white by thoroughly spraying same, and placed in a thermostatic chamber at 80°C for 5 hr. As a control, egg white was taken out by partly removing the eggshell of a chicken egg, the egg white thus taken out was placed in a petri dish, and the petri dish was placed in a thermostatic chamber at 80°C for 5 hr.

As a result, aggregation was not found in the egg white attached with the protein aggregation inhibitor obtained in Example 1, whereas the egg white free of the attachment of the protein aggregation inhibitor showed aggregation.

Therefore, it is clear that aggregation of protein contained in a chicken egg can be inhibited even when egg white of the chicken egg is heated, by using the protein aggregation inhibitor of the present invention.

As explained above, it is clear that the protein aggregation inhibitor of the present invention can effectively inhibit aggregation of protein due to heating. In addition, it is clear that a decrease in the activity of protein can be effectively inhibited even when the protein is heated, by using the protein aggregation inhibitor of the present invention.

### [Industrial Applicability]

The protein aggregation inhibitor of the present invention can inhibit aggregation of protein even when the protein is heat treated, and can further inhibit a decrease in the activity even when the protein is heated. Therefore, it is expected to be used, for example, for various applications where inhibition of proteins aggregation is desired such as preservative for enzyme, antibody drug, in vivo amyloid aggregation inhibitor and the like.

Therefore, the protein aggregation inhibitor of the present invention is expected to be used in various fields such as food processing field, antibody drug field, transplantation therapy field, enzyme production field, pharmaceutical-related field and the like.

## Claims

1. A protein aggregation inhibitor used for preventing protein aggregation and characteristically containing a sulfobetaine polymer obtained by polymerizing monomer components containing sulfobetaine monomer represented by the formula (I): 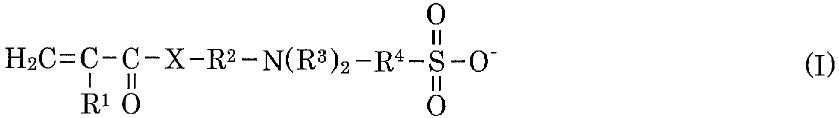 wherein R¹ is a hydrogen atom or a methyl group, R² is an alkylene group having a carbon number of 1 - 4, R³ is an alkyl group having a carbon number of 1 - 4, R⁴ is an alkylene group having a carbon number of 1 - 4, and X is an -NH- group or an - O- group.
